# DEMANDE DE BREVET EUROPEEN

(11) **EP 1 503 117 A1**
(43) Date de publication de la demande: **02.02.2005**
(21) Numéro de dépôt: 03077371.7
(22) Date de dépôt: 29.07.2003
(51) Int. Cl.: F16K 1/30, F16K 1/44, A61M 25/10, F17C 13/04

(54) **Robinet pour le soutirage de volumes de gaz limités d'une bouteillage de gaz**

(71) Demandeur: Luxembourg Patent Company S.A., 7440 Lintgen (LU)
(72) Inventeur: Kerger, Loll, 5613 Mondorf-les-Bains (LU)
(74) Mandataire: Weydert, Robert

(57) **Abrégé**

Le robinet pour le soutirage de volumes de gaz limités, notamment pour le gonflage de ballons, est pourvu d'un joint d'étanchéité flexible (38) monté sur une tige de commande manuelle (32) et ayant deux surfaces d'étanchéité dont chacune coopère avec un siège d'étanchéité (28, 30) formé sur le corps (12) du robinet (10). L'un des sièges d'étanchéité (28) est formé pour engager une surface d'étanchéité intérieure du joint d'étanchéité (38) et l'autre siège d'étanchéité (30) entourant le premier siège d'étanchéité (28) coopère avec une seconde surface d'étanchéité extérieure formée sur une lèvre circonférentielle flexible (38") du joint d'étanchéité (38). La lèvre flexible (38") est pourvue d'une ou plusieurs entailles radiales (39) en vue de limiter le débit du gaz dans la première phase d'ouverture du robinet (10) en mode de soutirage.

## Description

L'invention concerne un robinet à monter sur une bouteille de gaz et servant au soutirage de volumes de gaz limités, par exemple pour le gonflage de ballons.

Dans la technique antérieure un ensemble servant au soutirage de faibles quantités de gaz comportait un ensemble de robinet avec détendeur et buse de sortie pour le gaz soutiré. Cet ensemble est très complexe et ne peut pas être produit économiquement.

La présente invention a pour objet de remédier à ces désavantages et de réaliser un robinet du type précité d'une simple construction et qui peut être produit économiquement.

Pour résoudre cet objet il est conçu selon l'invention un robinet à double siège à monter sur une bouteille de gaz et servant au soutirage de volumes de gaz limités, par exemple pour le gonflage de ballons, comportant un corps de robinet formant un élément de robinet stationnaire, une tige de commande à actionner manuellement et formant un élément de robinet mobile, un premier siège de robinet prévu sur un desdits éléments de robinet et coopérant avec une première surface de joint d'étanchéité prévue sur l'autre desdits éléments de robinet, ladite première surface de joint d'étanchéité étant en engagement étanche avec le premier siège dans une position fermée du robinet et au moins partiellement écartée de celui-ci en position ouverte du robinet, un second siège de robinet prévu sur un desdits éléments de robinet et coopérant avec une seconde surface de joint d'étanchéité prévue sur l'autre desdits éléments de robinet, au moins un passage de soutirage formé dans la seconde surface de joint d'étanchéité ou dans le second siège, ce passage de soutirage permettant un débit de gaz limité entre le second siège et la seconde surface de joint d'étanchéité au moins pendant une première phase d'ouverture du robinet en mode de soutirage.

Ce robinet ne comporte que deux pièces principales, c'est-à-dire le corps de robinet et la tige de commande. Deux sièges de robinet sont prévus qui coopèrent avec deux surfaces de joint d'étanchéité. Le premier siège et la première surface de joint d'étanchéité servant de moyen d'ouverture/fermeture principal du robinet et le deuxième siège de robinet en coopération avec la deuxième surface de joint d'étanchéité servant de moyen d'ouverture/fermeture secondaire et de moyen de limitation de débit pour empêcher que le gaz s'échappe subitement en grande quantité à vitesse élevée en mode de soutirage. Le robinet selon l'invention est donc un robinet de soutirage à double siège. Un détendeur spécial n'est nécessaire.

Selon une caractéristique avantageuse du robinet à double siège selon l'invention les deux sièges et les deux surfaces d'étanchéité sont disposés et arrangés de sorte que, lors de la fermeture du robinet, le second siège en engageant la seconde surface d'étanchéité cause une flexion, en direction axiale du robinet, d'au moins une partie d'un joint d'étanchéité pourvu de cette seconde surface d'étanchéité.

Les deux sièges d'étanchéité sont de préférence concentriques et formés sur le corps du robinet et les deux surfaces de joint d'étanchéité sont de préférence concentriques et portées par la tige de commande.

Selon une autre caractéristique avantageuse du robinet selon l'invention le second siège est disposé radialement à l'extérieur du premier siège et le second siège est décalé du premier siège vers l'amont par rapport à la direction d'écoulement du gaz par le robinet en mode de soutirage, et la seconde surface d'étanchéité est disposée radialement à l'extérieur de la première surface d'étanchéité, et la seconde surface d'étanchéité est décalée de la première surface d'étanchéité vers l'amont par rapport à la direction d'écoulement du gaz par le robinet en mode de soutirage.

Le passage de soutirage peut être formé dans la seconde surface d'étanchéité par une entaille coupée dans la circonférence externe d'un joint d'étanchéité pourvu de cette seconde surface d'étanchéité. Au lieu de cette entaille le passage de soutirage peut être formé par un orifice.

Selon un mode d'exécution préféré du robinet selon l'invention la première et la seconde surface de joint d'étanchéité sont formées sur le même joint d'étanchéité. Dans ce cas le joint d'étanchéité est pourvu d'une lèvre flexible circonférentielle pourvu de la seconde surface d'étanchéité et ayant une plus faible épaisseur qu'une partie du joint d'étanchéité pourvu de la première surface d'étanchéité et se trouvant radialement à l'intérieur de cette lèvre, dans la position fermée du robinet le premier siège est en engagement avec la partie plus épaisse du joint d'étanchéité et le second siège est en engagement avec la lèvre et la fléchit vers l'amont par rapport à la direction d'écoulement du gaz par le robinet en mode de soutirage, et ledit au moins un passage de soutirage comporte un entaille ou un orifice formé dans la lèvre flexible.

Encore d'autres caractéristiques avantageuses de l'invention sont définies dans les revendications dépendantes.

L'invention sera maintenant expliquée en plus grand détail en référence aux dessins annexés sur lesquels :
La figure 1 montre le robinet à double siège selon l'invention raccordé à une bouteille de gaz.
La figure 2 montre le robinet selon l'invention en coupe longitudinale et en position de soutirage.
La figure 3 représente le joint d'étanchéité en coupe radiale et en vue de dessus.
La figure 4 est un détail agrandi de la figure 2 montrant la position ouverte du robinet en mode de soutirage.
4
La figure 5 représente le robinet à double siège en coupe verticale en position ouverte pour le remplissage de la bouteille de gaz.
La figure 6 représente un détail du robinet en position de remplissage.
La figure 7 montre une vue partielle du robinet en position fermée.

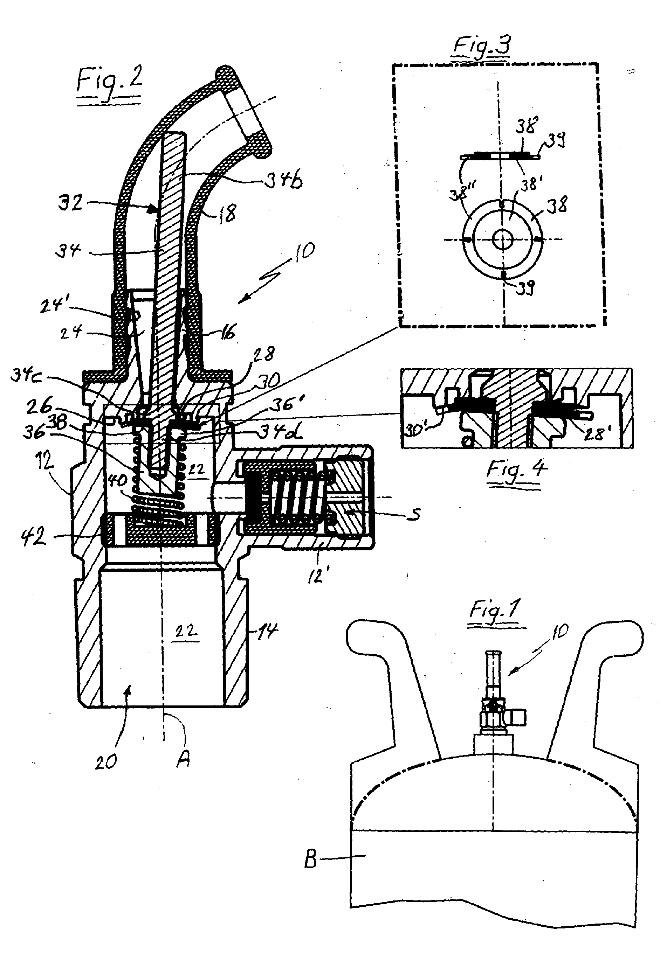

Revenons à la figure 1, le robinet 10 à double siège représenté en partie en élévation et en partie en coupe est fixé à une bouteille de gaz B.

La figure 2 montre le robinet 10 en coupe longitudinale et en position ouverte pour le soutirage d'un petit volume de gaz de la bouteille de gaz B, par exemple en vue du gonflage d'un ballon. Le robinet 10 comporte un corps de robinet 12 ayant une extrémité filetée inférieure 14 (le filetage n'est pas représenté) pour raccordement à la bouteille de gaz B. A son extrémité supérieure le corps du robinet 12 comporte un embout de sortie ou raccord de tuyau 16 à diamètre réduit sur lequel un manchon flexible et annulaire 18 en caoutchouc ou n'importe quelle autre matière flexible appropriée est monté. Ce manchon 18 flexible forme un tube de sortie flexible du gaz et prolonge l'embout de sortie 16.

Le corps 12 du robinet 10 est pourvu d'un passage axial longitudinal 20 à différents diamètres et comportant une partie inférieure à grand diamètre 22 et une partie supérieure à petit diamètre 24. Entre ces deux parties à grand et petit diamètre 22 et 24 se trouve une épaule radiale 26 pourvue de deux sièges de robinet annulaires concentriques 28 et 30 écartés l'un de l'autre en direction radiale et terminant en différentes positions axiales le long de l'axe longitudinal A du robinet. Les deux sièges de robinet 28 et 30 ont chacune une surface conique annulaire 28' et 30' faisant face vers l'extrémité de raccordement 14 du robinet 10 à la bouteille de gaz. La surface conique 30' du siège externe 30 a un angle de conicité inférieur à 180° et l'angle de conicité de la surface conique 28' du premier siège 28 est supérieur à 180°. En haut de l'épaule radiale 26 la partie à petit diamètre 24 a une partie conique 24' qui s'évase vers l'extrémité supérieure du raccord de tuyau 16. Cette partie conique 24' s'étendant approximativement le long de la longueur du raccord de tuyau 16.

Entre les extrémités supérieure et inférieure du corps 12 du robinet 10 se trouve une partie de corps 12' s'étendant radialement et renfermant une soupape de sécurité S de n'importe quelle construction connue et dont la construction n'est pas essentielle pour la compréhension de la présente invention. Cette soupape de sécurité 5 ne sera donc pas décrite ici en détail.

Le robinet à double siège 10 est aussi pourvu d'une tige de commande manuelle 32 disposée en partie dans le passage axial 22 et comportant une partie principale allongée 34 s'étendant par la partie conique 24' de la partie à petit diamètre 24 du passage 20 de sorte que son extrémité inférieure 34a se trouve dans la partie à grand diamètre 22 du passage 20 et son extrémité de commande supérieure 34b s'étend vers le haut par le raccord de tuyau 16 hors du corps de robinet 12 et se trouve à l'intérieur du manchon flexible 18. La partie principale 34 a dans sa partie inférieure 34a une épaule radiale 34c et en bas de cette épaule radiale 34c une partie filetée 34d. Vissé sur cette partie filetée 34d est un capuchon 36 qui sert à monter un joint d'étanchéité 38 par serrage de ce joint 38 entre le capuchon 36 et l'épaule radiale 34c de la partie principale 34 de la tige de commande 32.

Un ressort hélicoïdal 40 qui s'appuie à son extrémité inférieure sur un support 42 prévu dans la partie à grand diamètre 22 du passage 20 entoure du capuchon 36 et engage à son extrémité opposée une tête 36' prévue à l'extrémité supérieure du capuchon 36 en vue de forcer la tige de commande 32 vers le haut en position fermée du robinet.

Le joint d'étanchéité 38 est représenté sur la figure 3 en coupe radiale et en vue de dessus. Ce joint d'étanchéité 38 est pourvu d'une partie interne 38' et d'une lèvre circonférentielle flexible 38" s'étendant tout autour de la partie interne 38' et ayant une plus faible épaisseur que la partie interne 38'. Plusieurs entailles 39 espacées circonférentiellement l'une de l'autre sont coupées dans la circonférence externe de la lèvre flexible 38". Selon le mode d'exécution représenté quatre de ces entailles 39 sont prévues mais il pourrait y en avoir moins de quatre ou davantage. Une seule entaille 39 suffit. La partie interne épaisse 38' est pourvue en son centre d'un trou par lequel la partie filetée 34d de la partie principale 34 de la tige de commande 32 s'étend.

En position fermée, représentée en figure 7, du robinet à double siège, la surface supérieure de la partie épaisse interne 38' du joint d'étanchéité 38 formant une première surface de joint d'étanchéité est en engagement étanche avec le siège d'étanchéité annulaire interne 28 et la surface supérieure de la lèvre flexible 38" formant une seconde surface de joint d'étanchéité 38 est en engagement étanche avec le siège annulaire externe 30. Dans cette position la lèvre flexible 38" est légèrement fléchie vers le bas (c'est-à-dire vers l'amont dans la direction d'écoulement du gaz par le robinet en mode de soutirage) par le siège annulaire externe 30 dont la surface conique annulaire 30' est située un peu plus bas que la surface conique annulaire 28' du siège annulaire interne 28.

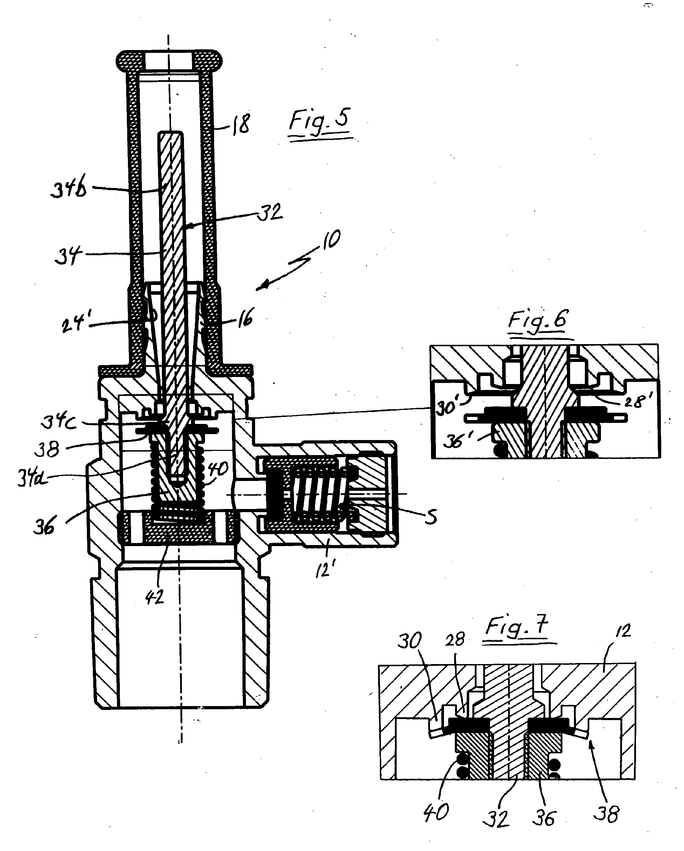

Pour soutirer du gaz de la bouteille de gaz B l'extrémité supérieure du manchon flexible 18 est saisie par la main et déformée ou fléchie latéralement vers un côté. Cette déviation provoque une inclinaison de la tige de commande 32, comme représenté sur la figure 2 jusqu'à ce qu'elle vienne buter contre la surface conique 24' de la partie supérieure à petit diamètre 24 du passage 20. Cette inclinaison de la tige de commande 32 cause le dégagement de la partie centrale épaisse 38' du joint 38 du siège de robinet interne 28 sur au moins une partie de sa circonférence et un dégagement limité de la surface supérieure de la lèvre flexible 38" du siège de robinet externe 30, comme représenté en figure 4, permettant un faible débit de gaz entre le côté supérieur de la lèvre flexible 38" et le siège externe 30 et entre le côté supérieur de la partie épaisse interne 38' du joint d'étanchéité 38 et le siège interne 28. Même si pendant le premier mouvement d'inclinaison de la tige de commande 32 la lèvre flexible 38" reste d'abord encore en contact avec le siège externe 30, au moins une des entailles 39 permet déjà un débit de gaz très limité. Au début, l'opérateur peut tourner le manchon flexible fléchi légèrement dans l'une ou l'autre direction en vue de trouver une des entailles 39 qui permet le débit initial très limité. Si l'opérateur veut augmenter le débit du gaz il peut pousser la tige de commande 32 au moyen du manchon flexible 18 vers le bas afin de dégager le joint d'étanchéité 38 davantage des deux sièges 28 et 30.
Il est noté qu'au début de l'ouverture du robinet 10, le joint 38 est dégagé du siège principal intérieur 28 alors que le joint 38, à cause de l'état fléchi de la lèvre élastique 38", reste encore en engagement avec le second siège externe 30 et le débit est initialement limité par l'une ou l'autre, ou plusieurs, des entailles 39. En d'autres mots, le robinet 10 est conçu de sorte qu'à la première ouverture du robinet 10, le moyen d'ouverture/fermeture principal (premier siège interne 28 et partie centrale épaisse 38' du joint 38) ouvre d'abord alors que le moyen d'ouverture fermeture secondaire (second siège externe 30 et lèvre externe flexible 38") reste encore plus ou moins fermé, à l'exception d'au moins une des entailles 39 qui limite le débit.

Le second siège 30 est décalé axialement du premier siège 28 vers l'amont par rapport à la direction d'écoulement du gaz en mode de soutirage (c'est-à-dire vers l'extrémité de raccordement du robinet 10 à la bouteille de gaz B) et la seconde surface d'étanchéité est décalée similairement de la première surface d'étanchéité, mais le décalage des deux sièges 28, 30 peut être plus élevé que le décalage des deux surfaces d'étanchéité de sorte qu'à la fermeture de robinet 10 le second siège 30 engage d'abord la lèvre 38" et la fléchit vers le bas avant que le premier siège 28 n'engage la partie centrale 38' du joint 38. De cette façon la flexion est augmentée en vue d'obtenir un très faible débit de gaz au début de l'ouverture du robinet.

Lorsque le manchon flexible est relâché il reprend tout de suite, sur l'influence de son élasticité, sa position initiale non-fléchie représentée sur la figure 5 et le ressort hélicoïdal cause instantanément la fermeture du robinet 10, c'est-à-dire l'engagement du joint d'étanchéité 38 avec les deux sièges 28 et 30.

La figure 5 représente la position de remplissage de la bouteille de gaz B. Dans cette position de manchon flexible 18 n'est pas déformé latéralement mais, par tout moyen approprié, une force axiale est exercée à la tige de commande 32 en vue de la déplacer vers une position à ouverture maximale dans laquelle le joint d'étanchéité 38 est dégagé totalement des deux sièges de robinet 28 et 30 pour permettre le remplissage rapide de la bouteille B.

Au lieu du mode d'exécution préférentiel représenté, le joint d'étanchéité pourrait être porté par le corps 10 du robinet et les deux sièges 28, 30 pourraient être prévus sur la tige de commande 32. En outre, au lieu des entailles 39 plusieurs petits orifices circulaires ou d'une autre forme appropriée pourrait être prévus à proximité de la circonférence externe de la lèvre flexible 38".

Selon un autre mode d'exécution, au lieu des entailles 39 le joint d'étanchéité 38 pourrait être dépourvu d'entailles et des petites rainures de soutirage (non-représentées) pourraient être prévues dans la surface conique annulaire 30' du siège externe 30.

Finalement, au lieu d'avoir un joint d'étanchéité unique ayant une partie épaisse interne 38' et une lèvre flexible externe 38" à épaisseur réduite, deux joints d'étanchéité séparés à diamètres différents, par exemple empilés l'un sur l'autre, pourraient être prévus. Selon encore un autre mode d'exécution une surface d'étanchéité peut être prévue sur la tige de commande et coopérant avec un premier siège de robinet sur le corps du robinet et une seconde surface d'étanchéité peut être prévue sur le corps du robinet coopérant avec un second siège de robinet sur la tige de commande. Aussi dans ce mode d'exécution les sièges et les surfaces d'étanchéité peuvent être disposés et arrangés pour fléchir, en position fermée du robinet, au moins la partie du joint d'étanchéité portant la seconde surface d'étanchéité.

D'autres modifications peuvent être apportées par l'homme de l'art au robinet à double siège décrit dans la description précédente sans pour cela quitter le cadre de l'invention, tel que défini par la portée des revendications annexées.

## Revendications

1. Robinet à double siège à monter sur une bouteille de gaz et servant au soutirage de volumes de gaz limités, par exemple pour le gonflage de ballons, comportant un corps de robinet formant un élément de robinet stationnaire, une tige de commande à actionner manuellement et formant un élément de robinet mobile, un premier siège de robinet prévu sur un desdits éléments de robinet et coopérant avec une première surface de joint d'étanchéité prévue sur l'autre desdits éléments de robinet, ladite première surface de joint d'étanchéité étant en engagement étanche avec le premier siège dans une position fermée du robinet et au moins partiellement écartée de celui-ci en position ouverte du robinet, un second siège de robinet prévu sur un desdits éléments de robinet et coopérant avec une seconde surface de joint d'étanchéité prévue sur l'autre desdits éléments de robinet, au moins un passage de soutirage formé dans la seconde surface de joint d'étanchéité ou dans le second siège, ce passage de soutirage permettant un débit de gaz limité entre le second siège et la seconde surface de joint d'étanchéité au moins pendant une première phase d'ouverture du robinet en mode de soutirage.

2. Robinet selon la revendication 1, **caractérisé en ce que** les deux sièges et les deux surfaces d'étanchéité sont disposés et arrangés de sorte que, lors de la fermeture du robinet, le second siège en engageant la seconde surface d'étanchéité cause une flexion, en direction axiale du robinet, d'au moins une partie d'un joint d'étanchéité pourvu de cette seconde surface d'étanchéité.

3. Robinet selon la revendication 1 ou 2, **caractérisé en ce que** les deux sièges d'étanchéité sont formés sur le corps du robinet et les deux surfaces de joint d'étanchéité sont portées par la tige de commande.

4. Robinet selon l'une quelconque des revendications 1 à 3, **caractérisé en ce que** les deux sièges et les deux surfaces d'étanchéité sont concentriques.

5. Robinet selon l'une des revendications 1 à 4, **caractérisé en ce que** le second siège est disposé radialement à l'extérieur du premier siège et le second siège est décalé du premier siège vers l'amont par rapport à la direction d'écoulement du gaz par le robinet en mode de soutirage, et la seconde surface d'étanchéité est disposée radialement à l'extérieur de la première surface d'étanchéité, et la seconde surface d'étanchéité est décalée de la première surface d'étanchéité vers l'amont par rapport à la direction d'écoulement du gaz par le robinet en mode de soutirage.

6. Robinet selon l'une quelconque des revendications 1 à 5, **caractérisé en ce que** ledit au moins un passage de soutirage est formé dans la seconde surface d'étanchéité par une entaille coupée dans la circonférence externe d'un joint d'étanchéité pourvu de cette seconde surface d'étanchéité .

7. Robinet selon l'une quelconque des revendications 1 à 5, **caractérisé en ce que** ledit au moins un passage de soutirage est formé dans la seconde surface d'étanchéité par un orifice pratiqué à proximité de la circonférence externe d'un joint d'étanchéité pourvu de cette seconde surface d'étanchéité.

8. Robinet selon l'une quelconque des revendications précédentes, **caractérisé en ce que** la première et la seconde surface de joint d'étanchéité sont formées sur le même joint d'étanchéité.

9. Robinet selon la revendication 8, **caractérisé en ce que** le joint d'étanchéité est pourvu d'une lèvre flexible circonférentielle pourvu de la seconde surface d'étanchéité et ayant une plus faible épaisseur qu'une partie du joint d'étanchéité pourvu de la première surface d'étanchéité et se trouvant radialement à l'intérieur de cette lèvre, et **en ce que** dans la position fermée du robinet le premier siège est en engagement avec la partie plus épaisse du joint d'étanchéité et le second siège est en engagement avec la lèvre et la fléchit vers l'amont par rapport à la direction d'écoulement du gaz par le robinet en mode de soutirage.

10. Robinet selon la revendication 9, **caractérisé en ce que** ledit au moins un passage de soutirage comporte une entaille ou un orifice formé dans la lèvre flexible.

11. Robinet selon l'une quelconque des revendications précédentes, **caractérisé par** un moyen de ressort en vue de solliciter la tige de commande vers sa position fermée.

12. Robinet selon l'une quelconque des revendications précédentes, **caractérisé en ce qu'**un manchon tubulaire en matière élastique formant tube de sortie du gaz en mode de soutirage est fixé à un embout de sortie du corps du robinet en vue de le prolonger, une partie de la tige de commande s'étendant par cet embout hors du corps de robinet et étant reçue avec jeu radial à l'intérieur dudit manchon élastique, ladite tige pouvant être déplacée manuellement au moyen du manchon tubulaire par rapport au corps du robinet en vue d'ouvrir le robinet.

13. Robinet selon la revendication 12, **caractérisé en ce que** l'embout de sortie a un passage interne conique limitant un mouvement d'inclinaison de la tige de commande en vue de l'ouverture du robinet dans un mode de soutirage.

14. Robinet selon l'une quelconque des revendications 1 à 13, **caractérisé en ce que** les deux sièges de robinet ont chacune une surface conique, la surface conique annulaire du second siège ayant un angle de conicité inférieur à 180° et l'angle de conicité de la surface conique du premier siège étant supérieur à 180°.
